Europäisches Patentamt
European Patent Office
Office européen des brevets

(19) (11) Veröffentlichungsnummer: 0 205 069 A2

(12) EUROPÄISCHE PATENTANMELDUNG

(21) Anmeldenummer: 86107487.0

(22) Anmeldetag: 02.06.86

(51) Int. Cl.⁴: C07D 513/04 , B41M 5/12 , C07D 277/42 , C07D 277/40 , B41M 5/26 , //(C07D513/04,311:00,277:00)

(30) Priorität: 14.06.85 DE 3521406

(43) Veröffentlichungstag der Anmeldung: 17.12.86 Patentblatt 86/51

(84) Benannte Vertragsstaaten: CH DE FR GB LI

(71) Anmelder: BAYER AG
Konzernverwaltung RP Patentabteilung
D-5090 Leverkusen 1 Bayerwerk(DE)

(72) Erfinder: Heidenreich, Holger, Dr.
Andreas-Gryphius-Strasse 22
D-5000 Köln 80(DE)
Erfinder: Wolfrum, Gerhard, Dr.
Domblick 17
D-5090 Leverkusen 3(DE)
Erfinder: Nehen, Ulrich, Dr.
Rückertstrasse 10
D-5090 Leverkusen 1(DE)

# (54) 2,2-Diaryl-chromenothiazole, ihre Herstellung und ihre Verwendung in Aufzeichnungsmaterialien.

(57) 2,2-Diarylchromenothiazole der allgemeinen Formel

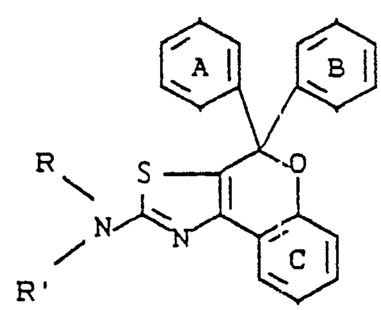

(I)

worin

R und R' unabhängig voneinander Wasserstoff, Alkyl, Cycloalkyl, Aralkyl bedeuten oder

R und R' ringgeschlossen sind und die Reste und die Ringe A, B und C ihrerseits in der Farbstoffchemie übliche nichtionische Substituenten tragen können, und mit einem Benzolring annelliert sein können,

sowie die Verwendung dieser Verbindungen als Farbbildner in einem druck-oder wärmeempfindlichen Aufzeichnungsmaterial.

EP 0 205 069 A2

# 2,2-Diaryl-chromenothiazole, ihre Herstellung und ihre Verwendung in Aufzeichnungsmaterialien

Gegenstand der Erfindung sind 2,2-Diaryl-chromenothiazole der allgemeinen Formel

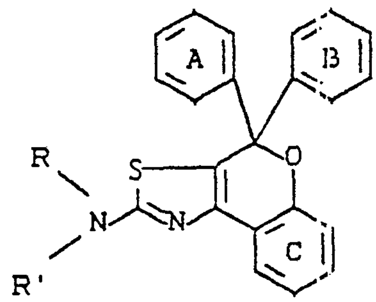

(I)

worin

R und R' unabhängig voneinander Wasserstoff, Alkyl, Cycloalkyl, Aralkyl bedeuten oder

R und R' ringgeschlossen sind und die Reste und die Ringe A, B und C ihrerseits in der Farbstoffchemie übliche nichtionische Substituenten tragen können, und mit einem Benzolring annelliert sein können,

ihre Herstellung und ihre Verwendung in druckkopierfähigen und thermoreaktiven Aufzeichnungsmaterialien.

In der Farbchemie übliche nichtionische Substituenten sind z.B.: Halogen, Hydroxy, Alkoxy, Aryloxy, Aralkoxy, Aryl, Cycloalkyl, Hetaryl, Alkylmercapto, Arylmercapto, Aralkylmercapto, Alkylsulfonyl, Cyan, Alkylcarbonyl, Alkylcarbonyloxy, Carbamoyl, Alkoxycarbonyl, Amino, das durch 1 oder 2 Alkyl-, Aryl-oder Aralkylgruppen substituiert sein kann, oder dessen Substituenten ringgeschlossen sein können, Acylamino, Alkenyloxy, Alkylcarbonyloxy und Arylcarbonyloxy und als Substituenten der Ringe außerdem Alkyl, Aralkyl, Nitro, Alkenyl oder Arylvinyl.

Bevorzugt steht Alkyl für $C_1$-$C_{22}$-Alkyl, insbesondere für $C_1$-$C_{12}$-Alkyl und ganz besonders für $C_1$-$C_6$-Alkyl und Alkenyl für $C_2$-$C_5$-Alkenyl.

Unter Halogen ist im besonderen Fluor, Chlor und Brom zu verstehen.

Insbesondere werden unter Cycloalkyl, Cyclopentyl und Cyclohexyl, unter Aryl Phenyl und Naphthyl, unter Aralkyl Benzyl und Phenethyl, und unter Hetaryl Pyridyl, Pyrimidyl, Pyrazinyl, Triazinyl, Imidazolyl, Oxazolyl, Thiazolyl, Triazolyl, Thiadiazolyl oder Tetrazolyl verstanden.

Aminogruppen, deren Substituenten ringgeschlossen sind, z.B. die Gruppe NRR', stehen bevorzugt für einen 5-oder 6-gliedrigen Ring wie Pyrrolidin, Pyrazolin, Piperidin, Piperazin und Morpholin.

Bevorzugtes Acyl ist $C_1$-bis $C_4$-Alkylcarbonyl und -sulfonlyl und Benzoyl.

Von den Verbindungen der Formel (I) sind die Verbindungen der Formel

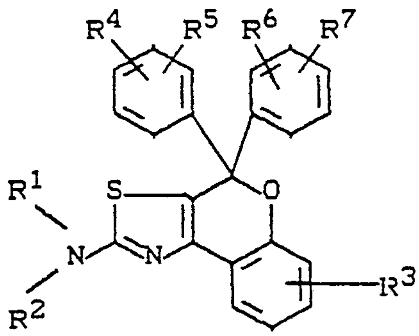

(II)

bevorzugt, worin

$R^1$ und $R^2$ unabhängig voneinander Wasserstoff, $C_1$-bis $C_{20}$-Alkyl, das substituiert sein kann durch Halogen, Cyano, $C_1$-bis $C_{20}$-Alkoxycarbonyl, $C_1$-bis $C_{20}$-Alkoxy, Hydroxy, Amino, das seinerseits durch ein oder zwei $C_1$-bis $C_{20}$-Alkyl-, Phenyl-oder Benzylgruppen substituiert sein kann, Cyclohexyl, Phenyl, Benzyl oder Phenylethyl, die substituiert sein können durch Halogen, Hydroxy, $C_1$-bis $C_{18}$-Alkyl, $C_1$-bis $C_{18}$-Alkoxy, Phenoxy, Benzyloxy, Phenyl, Biphenyl, $C_1$-bis $C_{18}$-Alkyl-sulfonyl, Phenylsulfonyl, Cyano oder Amino, das seinerseits durch 1 oder 2 $C_1$-bis $C_{18}$-Alkyl, Phenyl oder Benzyl substituiert sein kann,

oder

$NR^1R^2$ einen Pyrrolidin-, Pyrazolin-, Piperidin-, Piperazin-oder Morpholinrest, die durch $C_1$-bis $C_4$-Alkyl oder Phenyl substituiert sein können,

$R^3$ Wasserstoff, $C_1$-bis $C_{22}$-Alkyl, Cyclohexyl, Nitro, gegebenenfalls durch $C_1$-bis $C_{12}$-Alkyl, $C_1$-bis $C_4$-Alkoxy oder Chlor substituiertes Phenyl oder Benzyl,

$R^4$ und $R^6$ unabhängig voneinander Wasserstoff, $C_1$-bis $C_{22}$-Alkyl, $C_1$-bis $C_{22}$-Alkoxy oder Halogen,

$R^5$ und $R^7$ unabhängig voneinander Wasserstoff, $C_1$-bis $C_{22}$-Alkyl, $C_1$-bis $C_{22}$-Alkoxy, $C_1$-bis $C_{22}$-Alkylthio, Hydroxy, Halogen, $C_2$-bis $C_6$-Alkenyloxy, Phenyl, Phenoxy, Cyclohexyloxy, Benzyloxy, Phenylethoxy oder Phenylthio, sowie die genannten Ringe durch $C_1$-bis $C_4$-Alkyl, $C_1$-bis $C_4$-Alkoxy oder Halogen substituiert sein können, oder $-NR^1R^2$ bedeuten oder

$R^4$ oder $R^6$ zusammen mit dem Benzolring, an den sie gebunden sind, einen Naphthalinring darstellen.

Von besonderem Interesse sind Verbindungen der Formel

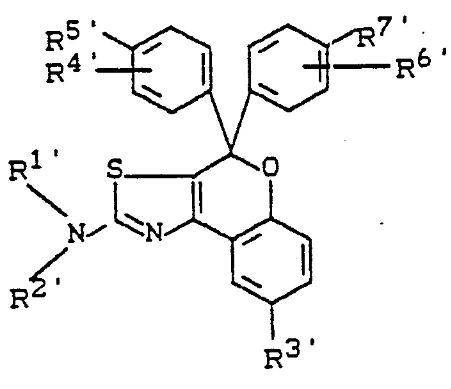

(III)

worin

$R^{1'}$ und $R^{2'}$ unabhängig voneinander Wasserstoff, $C_1$-bis $C_6$-Alkyl, das substituiert sein kann durch Cyano, Hydroxy, $C_1$-bis $C_3$-Alkoxycarbonyl oder $C_1$-bis $C_4$-Alkoxy, Benzyl oder Phenyl, die substituiert sein können durch Halogen, Hydroxy, $C_1$-bis $C_{12}$-Alkyl oder $C_1$-bis $C_4$-Alkoxy, Cyclohexyl, das durch $C_1$-bis $C_4$-Alkyl substituiert sein kann, oder

$NR^{1'}R^{2'}$ einen Pyrrolidin-, Pyrazolin-, Piperidin-, Piperazin-oder Morpholinrest, die durch Methyl oder Phenyl substituiert sein können,

$R^{3'}$ Wasserstoff, $C_1$-bis $C_{12}$-Alkyl, Phenyl oder Benzyl,

$R^{4'}$ und $R^{6'}$ unabhängig voneinander Wasserstoff, $C_1$-bis $C_{12}$-Alkyl, $C_1$-bis $C_4$-Alkoxy oder Chlor und

$R^{5'}$und $R^{7'}$ unabhängig voneinander Wasserstoff, $C_1$-bis $C_{12}$-Alkyl, $C_1$-bis $C_4$-Alkoxy, Phenoxy, Benzyloxy oder $-NR^{1'}R^{2'}$

bedeuten.

In besonders bevorzugten Verbindungen der Formel (III) stehen

$R^{1'}$ für $C_1$-bis $C_6$-Alkyl,

$R^{2'}$ für $C_1$-bis $C_6$-Alkyl oder Cyclohexyl,

$R^{3'}$ für Wasserstoff oder Methyl,

$R^{4'}$ und $R^{6'}$ für Wasserstoff,

$R^{5'}$ für Methoxy, Di-$C_1$-bis $C_6$-alkylamino oder Cyclohexyl-$C_1$-bis $C_6$-alkylamino und

$R^{7'}$ für Di-$C_1$-bis $C_6$-alkylamino oder Cyclohexyl-$C_1$-bis $C_6$-alkylamino.

Die Chromenothiazole der Formel (I) erhält man vorzugsweise durch Umsetzung einer Thiazol-Verbindung der Formel (IV) oder ihres Salzes der Formel (V)

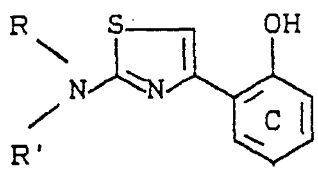

(IV)

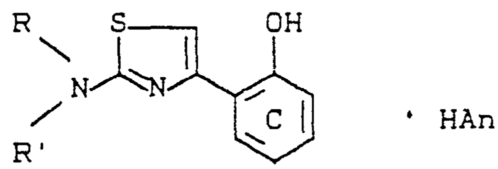

(V)

mit einer Carbinolverbindung der Formel

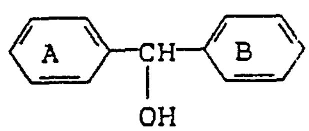

(VI)

worin

R,R',A,B, und C die angegebene Bedeutung haben, und

An das Anion einer organischen Säure oder vorzugsweisee einer anorganischen Säure, z.B. das Chlorid-, Bromid-, Fluorid, Sulfat-, Phosphat-oder insbesondere Perchlorat-Ion bedeutet,

und anschließende Oxidation.

Die Reaktion wird vorzugsweise so durchgeführt, daß man zuerst die Reaktionskomponenten in einem organischen Lösungsmittel, das mit Wasser mischbar und inert gegenüber den Ausgangsstoffen ist, zur Reaktion bringt und dann nach Zugabe einer wäßrigen Lösung eines Alkalimetallcarbonats oder Alkalimetallhydroxids das Umsetzungsprodukt mit einem Oxidationsmittel zur Chromenoverbindung oxidiert.

Die Umsetzung verläuft vorzugsweise in Anwesenheit eines sauren Katalysators, z.B. einer niederen aliphatischen Carbonsäure wie Ameisensäure oder Essigsäure oder einer anorganischen Säure wie Salzsäure, Schwefelsäure, Phosphorsäure oder Perchlorsäure sowie auch Phosphoroxichlorid.

Die Umsetzung kann bei einer Temperatur von 10° bis 110° C, vorzugsweise 20 bis 40° C vorgenommen werden. Die Oxidation kann bei Temperaturen von 0° bis 100°C erfolgen. Vorteilhafterweise oxidiert man im Bereich zwischen Raumtemperatur (20 bis 25°C) und 100°C, vorzugsweise bei Raumtemperatur.

Als organische Lösungsmittel eignen sich zweckmäßigerweise niedrige aliphatische Alkohole, wie z.B. Methanol, Ethanol oder Isopropanol oder cyclische Ether, wie z.B. Dioxan oder Tetrahydrofuran, γ-Butyrolacton, Acetonitril, Dimethylsulfoxid, Dimethylformamid, Dimethylacetamid oder insbesondere N-Methylpyrrolidon.

Als Alkalimetallcarbonate kommen z.B. Kaliumcarbonat oder Natriumcarbonat, als Alkalimetallhydroxid in erster Linie Kaliumhydroxid oder Natriumhydroxid in Betracht.

Als Oxidationsmittel kommen beispielsweise Chromate, Bichromate, Chlorate, Chlorite, Peroxide, Mangandioxid, Bleidioxid, Chlor, Brom, molekularer Sauerstoff, Luft, Perborate, Permanganate, Wasserstoffperoxid, Chrloranil und insbesondere wasserlösliche Salze des Hexacyanoferrat-III in Frage. Als wasserlösliche Salze des Hexacyanoferrat-III eignen sich vor allem die des Kaliums und Natriums. Anstelle des handelsüblichen Trikaliumhexacyanoferrat-III können auch Lösungen verwendet werden, die durch Oxidation von Kaliumhexacyanoferrat-II in Gegenwart von Säuren mit Wasserstoffperoxid bei Raumtemperatur erhalten werden.

Die Menge an wasserlöslichem Salz des Hexacyanoferrat-III beträgt mindestens die stöchiometrisch erforderliche Menge. Vorzugsweise verwendet man das 1,0 bis 1,2-fache der stöchiometrisch erforderlichen Menge, d.h. 2,0 bis 2,4 Mol Hexacyanoferrat-III je Mol der Reaktionskomponenten.

Nach Beendigung der Oxidation erfolgt die Isolierung der Chromenothiazol-Verbindung, indem man das rohe Produkt aus der Reaktionsmischung durch Abfiltrieren entfernt und mit Wasser neutral wäscht. Die erhaltene Chromenothiazol-Verbindung wird dann über Kieselgelplatten oder dünnschichtchromatographisch und/oder durch Umkristallisation gereinigt.

Gegenstand der Erfindung sind auch Aminothiazol-Derivate der Formel (IV), ihre Salze der Formel (V) und ihre Herstellung aus Phenolen der Formel

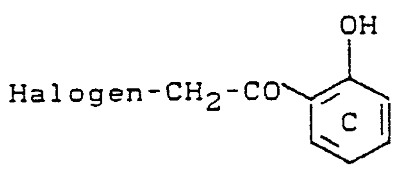

(VII)

mit Thioharnstoffen der Formel

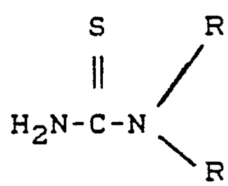

(VIII)

wobei das entstehende Halogenid durch ein anderes Anion ausgetauscht werden kann.

In den Formeln (IV), (V), (VII) und (VIII) haben die Substituenten R und R' und die möglichen Substituenten von C bevorzugt die bei den Formeln (II) und (III) angegebene Bedeutung.

Die Herstellung der Thioharnstoffe (VIII) wird von H. Hartmann in J. prakt. Chem. 315 (1973) S. 114-148 beschrieben.

Die erfindungsgemäßen Chromenothiazole (I) finden Verwendung als Farbbildner in druck-und wärmeempfindlichen Aufzeichnungsmaterialien. Sie sind normalerweise farblos oder höchstens - schwach gefärbt.

Wenn diese Farbbildner mit einem vorzugsweise sauren Entwickler, d.h. einem Elektronenakzeptor, in Kontakt gebracht werden, so ergeben sich intensive violette, blaue, grünblaue, blauschwarze oder grüne Farbtöne, die ausgezeichnete Echtheiten aufweisen. Sie zeigen auf phenolischen Unterlagen und besonders auf aktivierten Tonen als Entwickler eine gute Farbintensität, Sublimier-und Lichtechtheit.

Typische Beispiele für Entwickler sind anorganische Stoffe wie Tone, Metallsalze oder -oxide, Phenole, Phenolcarbonsäuren bzw. deren Ester oder organische Polymerisate wie Phenolharze und sauermodifizierte Polymere, z.B. sauermodifizierte Polyester, Polyamide und Polyacrylnitril.

Die Verbindungen (I) können auch im Gemisch mit anderen bekannten Farbbildnern, z.B. 3,3-Bis-(aminophenyl)-phthaliden, 3,3-Bis-(indolyl)-phthaliden, 3-Amino-fluoranen, 2,6-Diamino-fluoranen, Leukoauraminen, Spiropyranen, Spirodipyranen, Chromenoindolen, Phenoxazinen, Phenothiazinen, Carbazolylmethanen oder weiteren Triarylmethanleukofarbstoffen eingesetzt werden.

Die Verbindungen (I) eignen sich vor allem als Farbbildner in einem Aufzeichnungsmaterial, das sowohl Kopier-als auch Registriermaterial sein kann. Ihre Entwicklungsgeschwindigkeit ist in Abhängigkeit von den Substituenten unterschiedlich. Eine geringe Entwicklungsgeschwindigkeit führt zu einer reduzierten Empfindlichkeit der Aufzeichnungsmaterialien gegenüber unbeabsichtigter vorzeitiger Entwicklung.

Insbesondere beim Thermodruck-Verfahren sind mit den erfindungsgemäßen Farbbildnern Schriftbilder extrem hoher Echtheiten und hoher Unempfindlichkeit gegen den Einfluß sowohl saurer als auch basischer Medien erhältlich.

Ein druckempfliches Material besteht beispielsweise aus mindestens 1 Paar von Blättern, die mindestens einen Farbbildner der Formel (I), gelöst oder dispergiert in einem nichtflüchtigen organischen Lösungsmittel, und einen Elektronenakzeptor als Entwickler enthalten.

Der Farbbildner liefert an den Punkten, an denen er mit dem Elektronenakzeptor in Kontakt kommt, eine gefärbte Markierung. Um eine frühzeitige Aktivierung der Farbbildner zu verhindern, werden diese in der Regel von dem Elektronenakzeptor getrennt. Dies kann zweckmäßig erzielt werden, indem die Farbbildner in schaum-, -schwamm-oder bienenwabenartigen Strukturen eingearbeitet werden. Vorzugsweise sind die Farbbildner in Mikrokapseln eingeschlossen, die sich durch Druck zerbrechen lassen. Verfahren zur Herstellung derartiger Mikrokapseln sind bekannt.

Als nichtflüchtige Lösungsmittel sind z.B. partiell hydriertes Terphenyl, alkylierte Naphthaline oder Dibutylphthalat geeignet.

Bevorzugt wird eine Anordnung, bei der der eingekapselte Farbbildner in Form einer Schicht auf der Rückseite eines Übertragungsblattes und der Elektronenakzeptor in Form einer Schicht auf der Vorderseite eines Empfangsblattes vorhanden sind.

Eine andere Anordnung der Bestandteile besteht darin, daß die den Farbbildner enthaltenden Mikrokapseln und der Entwickler in oder auf dem gleichen Blatt in Form einer oder mehrerer Einzelschichten oder in der Papierpulpe vorliegen.

Die Verbindungen der Formel (I) können vorzugsweise auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel.

Thermoreaktive Aufzeichnungssysteme umfassen z.B. wärmeempfindliche Aufzeichnungs-und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Meßinstrumenten, wie z.B. Elektrokardiographen, verwendet. Die Bilderzeugung -(Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, daß der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, daß sowohl der Farbbildner als auch der Entwickler in einer Schicht dispergiert sind. Das Bindemittel wird in spezifischen Bereichen mittels Wärme erweicht und an diesen Stellen, an denen Wärme angewendet wird, kommt der Farbbildner mit dem Elektronenakzeptor in Kontakt, und es entwickelt sich sofort die erwünschte Farbe.

Als Entwickler eignen sich die gleichen Elektronenakzeptoren wie sie in druckempfindlichen Papieren verwendet werden, vorzugsweise phenolische Verbindungen, wie sie beispielsweise in der DE-PS 1 251 348 beschrieben sind, sowie Borsäure und organische, vorzugsweise aliphatische Dicarbonsäuren.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die 2,2-Diaryl-chromenothiazole und der Entwickler in Wasser schwerlöslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder -schmilzt das Bindemittel, so daß der Farbbildner mit dem Entwickler in Kontakt kommt und sich eine Farbe bilden kann. Wasserlösliche oder mindestens in Wasser quellbare Bindemittel sind z.B.

hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine und Stärke.

Die thermoreaktiven Schichten können weitere Zusätze enthalten: zur Verbesserung des Weißgrades, zur Erleichterung des Bedruckens der Papiere, zur Verhinderung des Festklebens der erhitzten Feder und zur Farbbildung nur innerhalb eines begrenzten Temperaturbereiches.

Die beschriebenen Verfahren und Zubereitungen sind beispielsweise aus den US-Patentschriften 2 948 753, 3 096 189 und 3 193 404 und aus den deutschen Offenlegungsschriften 2 555 080 und 2 700 937 bekannt.

Beispiel 1

In 250 ml N-Methylpyrrolidon löst man bei Raumtemperatur 17,15 g 2-Morpholino-4-(2'-hydroxyphenyl)-thiazol-hydrobromid und 8,5 g 4,4'-Bis-(dimethylamino)-benzhydrol. Man fügt 1 ml Perchlorsäure hinzu und läßt 5 Stunden nachrühren. Anschließend versetzt man mit 7 ml 40 %iger Natronlauge und oxidiert dann mit einer konzentrierten Lösung von 16,5 g Kaliumhexacyanoferrat-III in Wasser. Man verfolgt die Reaktion dünnschichtchromatographisch und arbeitet den Ansatz durch Einrühren in Eiswasser, dem etwas Ammoniak beigemischt wird, auf. Der Rückstand wird durch Umfällen aus DMF/Wasser gereinigt. Man erhält 25 g einer Verbindung der folgenden Formel

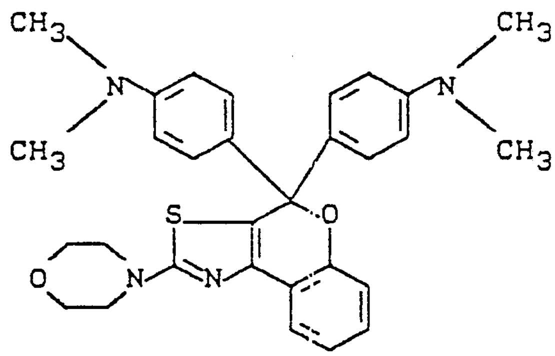

in Form eines farblosen Pulvers mit dem Schmelzpunkt: 198°C. Auf Säureton entwickelt dieser Farbbildner eine intensive blaue Farbe.

Das oben verwendete 2-Morpholino-4(2'-hydroxy-phenyl)-thiazol-hydrobromid wird folgendermaßen hergestellt: Zu einer siedenden Lösung von 73 g 1-Morpholino-thioharnstoff in 200 ml Ethanol läßt man eine Mischung von 107,5 g 2-Bromacetyl-phenol in 100 ml Ethanol tropfen. Nach 4 Stunden kühlt man den Reaktionsansatz ab und saugt den orangefarbenen Niederschlag ab. Das Hydrobromid schmilzt bei 255° C.

Beispiel 2

Zu einer Lösung von 19,15 g 2-(N-cyclohexyl-N-ethyl)-4-(2'-hydroxy-phenyl)-thiazol-hydrobromid und 8,5 g 4,4'-Bis-(dimethylamino)-benzhydrol in 250 ml Dimethyl-formamid gibt man 1 ml Perchlorsäure. Man läßt über Nacht bei Raumtemperatur rühren und oxidiert anschließend mit 16,5 g Kaliumhexacyanoferrat-III in der in Beispiel 1 beschriebenen Weise. Nach der Aufarbeitung erhält man 26,5 g einer Verbindung der Formel

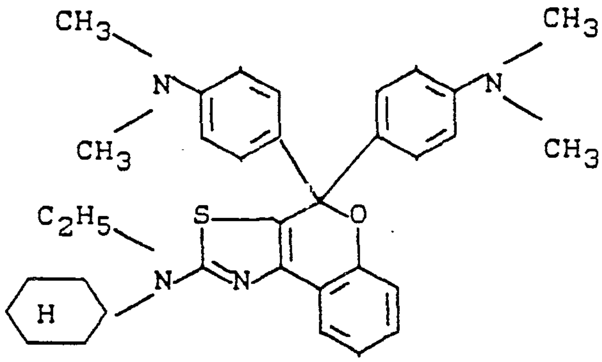

in Form eines grauen Pulvers mit dem Schmelzpunkt: 200° C. Auf Säureton entwickelt dieser Farbbildner eine intensive blaue Farbe.

Das für die obige Reaktion benötigte 2-(N-Cyclohexyl-N-ethyl)-4-(2'-hydroxy-phenyl)-thiazol-hydrobromid wird auf dem in Beispiel 1 gezeigten Wege hergestellt und schmilzt bei 155° C.

Nach Beispiel 1 können auch die folgenden Farbbildner hergestellt werden.

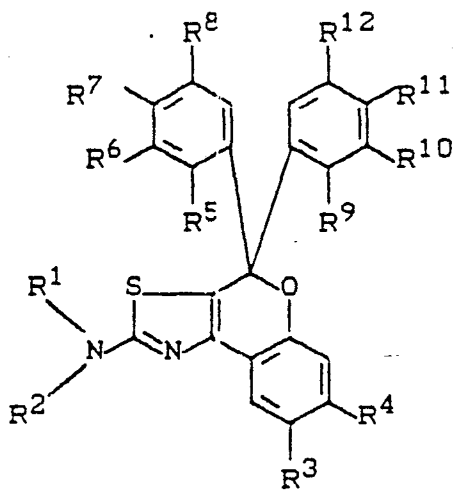

| Bsp. | $R^1$ | $R^2$ | $R^3$ | $R^4$ | $R^5$ | $R^6$ | $R^7$ | $R^8$ |
|---|---|---|---|---|---|---|---|---|
| 3 | $C_2H_5$ | $C_2H_5$ | H | H | H | H | $OCH_3$ | H |
| 4 | $C_4H_9$ | $C_4H_9$ | $CH_3$ | H | $OCH_3$ | H | $N(CH_3)_2$ | H |
| 5 | 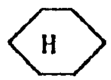 | $C_2H_5$ | H | H | $OCH_3$ | H | $N(C_2H_5)_2$ | $OCH_3$ |
| 6 | 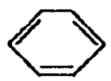 | $C_2H_5$ | H | $CH_3$ | Cl | H | $OCH_3$ | H |
| 7 | 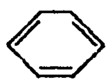 | H | H | H | H | $OCH_3$ | $N(C_3H_7)_2$ | H |
| 8 | 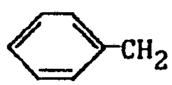 | 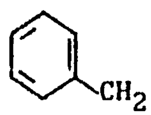 | $CH_3$ | H | $CH_3$ | H | 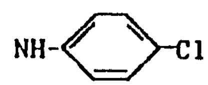 | H |
| 9 | $(CH_2)_5$ | | H | H | $CH_3$ | H | $OCH_3$ | $CH_3$ |
| 10 | $(CH_2)_4$ | | H | H | 2,3-Benzo | | $N(C_2H_5)_2$ | H |
| 11 | $C_2H_4CN$ | $CH_3$ | H | H | 2,3-Benzo | | $N(C_2H_5)$ | H |

<u>Fortsetzung Beispiele 3 bis 11</u>

| Bsp. | $R^9$ | $R^{10}$ | $R^{11}$ | $R^{12}$ | Farbton | Schmelzpunkt (°C) |
|---|---|---|---|---|---|---|
| 3 | H | H | $N(CH_3)_2$ | H | rotblau | 156-159 |
| 4 | $OCH_3$ | H | $N(CH_3)_2$ | H | blauschwarz | 142-145 |
| 5 | $OCH_3$ | H | $N(C_2H_5)_2$ | $OCH_3$ | blauschwarz | 187-190 |
| 6 | $CH_3$ | $CH_3$ | $N(C_4H_9)_2$ | $CH_3$ | violett | 200-202 |
| 7 | H | H | 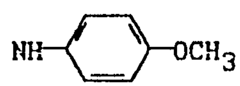 | H | blauschwarz | Öl |
| 8 | $CH_3$ | H | 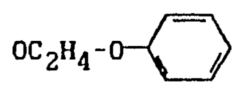 | H | dunkelblau | 242 |
| 9 | H | $OCH_3$ | $N(C_2H_5)_2$ | H | dunkelblau | 176-179 |
| 10 | Cl | H | $N(C_4H_9)_2$ | H | blaugrün | 223-228 |
| 11 | H | H | $OCH_3$ | H | schwarzviolett | 181-185 |

| Bsp. | $R^1$ | $R^2$ | $R^3$ | $R^4$ | $R^5$ | $R^6$ | $R^7$ | $R^8$ |
|---|---|---|---|---|---|---|---|---|
| 12 | $CH_3O$- 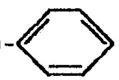 | H | H | H | Br | H | $OC_2H_5$ | H |
| 13 | 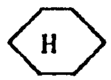 | $C_2H_5$ | H | H | H | $OCH_3$ | $OCH_3$ | $OCH_3$ |
| 14 | $C_4H_9$ | $C_4H_9$ | H | H | $OCH_3$ | H | H | $OCH_3$ |
| 15 | $C_2H_4OCH_3$ | $CH_3$ | H | H | 2,3-Benzo | | $NHC_2H_4CN$ | H |
| 16 | $C_2H_5$ | $C_2H_5$ | H | H | H | H | $OCH_3$ | H |
| 17 | $C_2H_4CN$ | $C_2H_4CN$ | $CH_3$ | H | $NO_2$ | H | $N(C_2H_5)_2$ | H |
| 18 | $C_3H_7$ | $C_3H_7$ | H | $CH_3$ | Cl | H | $NH-SO_2CH_3$ | Cl |
| 19 | $C_2H_4OAc$ | $C_2H_4OAc$ | H | H | $SCH_3$ | H | $N(C_2H_5)_2$ | $SCH_3$ |
| 20 | $C_4H_9$ | $CH_3$ | H | H | $NO_2$ | H | $NO_2$ | H |
| 21 | $C_2H_4CN$ | $C_2H_5$ | H | H | H | H | $NO_2$ | H |
| 22 | $CH_3$ | $CH_3$ | H | H | 2,3-Benzo | | NH- 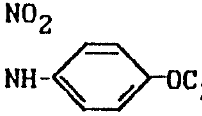 $-OC_2H_5$ | H |
| 23 | $C_2H_4OH$ | $C_2H_4OH$ | H | H | $OC_2H_5$ | H | $N(C_2H_5)_2$ | H |
| 24 | 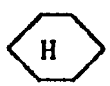 | $C_2H_5$ | H | H | H | H | 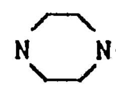 | H |
| 25 | $CH_2-CH_2-O-CH_2-CH_2$ | | H | H | H | $C_2H_5$ | NH- 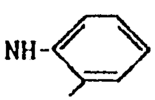 $C_2H_5$ | H |

Fortsetzung Beispiele 12 bis 25

| Bsp. | $R^9$ | $R^{10}$ | $R^{11}$ | $R^{12}$ | Farbton | Schmelzpunkt (°C) |
|---|---|---|---|---|---|---|
| 12 | $OC_2H_4OAc$ | H | $N(C_2H_4CN)_2$ | H | dunkelblau | Öl |
| 13 | H | H | $N(C_7H_7)_2$ | H | schwarzviolett | Öl |
| 14 | $CH_3$ | H | $N(C_2H_5)(C_2H_4CN)$ | $CH_3$ | rotblau | 140 (Zers.) |
| 15 | 2,3-Benzo | | $N(C_2H_5)_2$ | H | blaugrün | 157-160 |
| 16 | H | H | $OCH_3$ | H | orange | 138 |
| 17 | H | $OCH_3$ | $OCH_3$ | H | blauschwarz | 99-102 |
| 18 | 2,3-Benzo | | 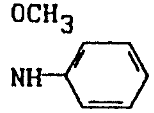 | H | blau | 200 |
| 19 | H | H | $N(C_2H_5)_2$ | H | blaugrün | 247-249 |
| 20 | H | H | 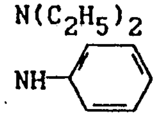 | H | grün | 170 (Zers.) |
| 21 | H | H | $N(C_2H_4OH)_2$ | H | graugrün | Öl |
| 22 | $CH_3$ | H | $OCH_3$ | H | blauschwarz | Öl |
| 23 | H | H | $NO_2$ | H | grün | 178 (Zers.) |
| 24 | $OCH_3$ | H | 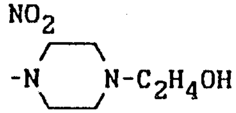 | H | dunkelblau | 270-275 |
| 25 | H | H | $N(C_2H_5)_2$ | H | graublau | 191 |

| Bsp. | $R^1$ | $R^2$ | $R^3$ | $R^4$ | $R^5$ | $R^6$ | $R^7$ | $R^8$ |
|---|---|---|---|---|---|---|---|---|
| 26 | $C_4H_9$ | $C_4H_9$ | H | H | $CH_3$ | H | $N(C_3H_7)_2$ | H |
| 27 | $C_4H_9$ | $CH_3$ | H | H | H | H | $OCH_3$ | H |
| 28 | 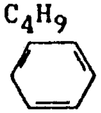 | $CH_3$ | H | H | H | H | 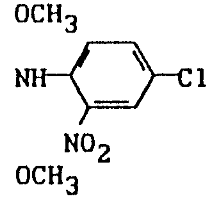 | H |
| 29 | $C_2H_5$ | $CH_3$ | H | $CH_3$ | H | H | $OCH_3$ | H |
| 30 | $i-C_3H_7$ | $i-C_3H_7$ | H | H | H | H | $OCH_3$ | H |
| 31 | $-CH_2-CH_2-\overset{CH_3}{\overset{\vert}{N}}-CH_2-CH_2-$ | | H | H | Cl | H | $N(C_2H_5)_2$ | $CH_3$ |
| 32 | " | | H | H | H | H | 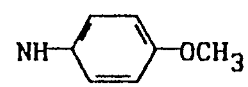 | H |
| 33 | $C_2H_5$ | $C_2H_5$ | $CH_3$ | H | NHAc | H | $N(C_2H_5)_2$ | H |
| 34 | 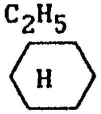 | $C_2H_5$ | H | H | H | H | $OCH_3$ | H |
| 35 | $C_2H_4CN$ | $CH_3$ | H | H | Cl | H | H | H |

Fortsetzung Beispiele 26 bis 35

| Bsp. | $R^9$ | $R^{10}$ | $R^{11}$ | $R^{12}$ | Farbton | Schmelzpunkt (°C) |
|---|---|---|---|---|---|---|
| 26 | $CH_3$ | H | $N(C_2H_5)C_2H_4N(CH_3)_2$ | H | blau | 231 (Zers.) |
| 27 | $CH_3$ | H | $N(C_2H_5)C_2H_4Cl$ | H | rotblau | 129-132 |
| 28 | H | H | $N(C_4H_9)_2$ | H | blauschwarz | 169 |
| 29 | H | H | 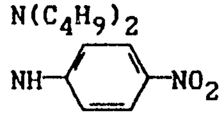 | H | dunkelblau | 130 (Zers.) |
| 30 | H | H | $N(C_2H_5)C_2H_4N(CH_3)_2$ | H | rotblau | Öl |
| 31 | H | H | 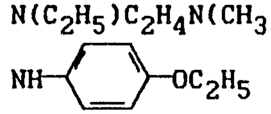 | H | blauschwarz | Öl |
| 32 | $CH_3$ | H | Cl | $CH_3$ | grüngrau | 112-115 |
| 33 | H | H | $OCH_3$ | H | schwarzgrün | 126-130 |
| 34 | H | H | 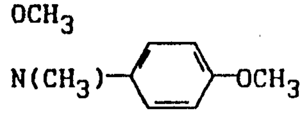 | H | dunkelgrün | Öl |
| 35 | $OCH_3$ | H | $NHC_4H_9$ | $OCH_3$ | schwarzviolett | 169-175 |

Beispiel 36: Herstellung eines kohlefreien Durchschreibepapiers

a.) Herstellung der Mikrokapseldispersion

10,0 g der Leukoverbindung, hergestellt nach Beispiel 1, werden unter Rühren und Erwärmen auf 80°C in 161,1 g eines diisopropylierten Diphenyls gelöst und nach Abkühlen auf Raumtemperatur nacheinander 36,75 g Oxadiazintriondi-hexamethylendiisocyanat (NCO-Gehalt 21,0 %) und 40,4 g Isohexadecan unter Rühren zugefügt.

Diese organische Lösung wird in 385 g einer 0,5 %igen, wäßrigen Lösung eines teilverseiften Polyvinylacetats (Verseifungsgrad 90 %) gegeben und an einer Mischsirene bei 15 700 U/min eine Emulsion mit einer Tröpfchengröße von 6-8 µm hergestellt. Zu dieser Emulsion werden unter Rühren 6,1 g Diethylentriamin gelöst in 61,8 g Wasser, zugefügt. Die gebildete 35 %ige Mikrokapseldispersion wird 20 Minuten bei 35°C und nach Aufheizen 2 Stunden bei 60°C nachgerührt.

b.) Herstellung des Durchschreibepapiers

Die in a.) hergestellte Mikrokapseldispersion wird mit Wasser auf einen Kapselanteil von 15 Gew.-% verdünnt und mit einer 30 µm Drahtrakel auf ein Rohpapier ( 50 g/m²)aufgestrichen.

Das Papier wird getrocknet und so ein Deckblatt eines kohlefreien Durchschreibepapiers hergestellt.

Eine Probe des Durchschreibepapiers wird mit der mit Mikrokapseln beschichteten Seite auf ein mit saurem Ton beschichtetes, handelsübliches Nehmerpapier (Giroset CF der Fa. Feldmühle) gelegt und 3 weitere Papiere aufgelegt. Mit einer Schreibmaschine wird dieser Papierset mit dem Buchstaben "w" beschriftet. Auf dem Nehmerpapier wird ein echter Durchschlag des "w" in einem intensiven blauen Farbton sichtbar.

Beispiel 37: Herstellung eines wärmeempfindlichen Aufzeichnungsblattes

In einer Kugelmühle werden 3,6 g eines Esterwachses mit einem Tropfpunkt von 79 -85°C - (Hoechst-Wachs E der Hoechst AG), 41 g Kaolin, 18 g eines teilverseiften Polyvinylalkohols (Mowiol 26-88 der Hoechst AG), 32 g Bis-(4-hydroxyphenyl)-dimethylmethan und 500 g Wasser sorgfältig gemahlen, bis die Teilchengröße ca. 10 µm erreicht ist.

Mit Hilfe eines Ultra-Turrax-Mischers werden 10,0 g des Leukofarbstoffs, der nach Beispiel 2 hergestellt wurde, 3 g eines teilverseiften Polyvinylalkohols (Mowiol 26 -88 der Hoechst AG) und 60 g Wasser bei 20,000 U/Min. intensiv vermischt, bis der Farbstoff in feiner Verteilung vorliegt. Der sich dabei bildende Schaum wird durch einige Tropfen Tributylphosphat zerstört.

Die beiden Dispersionen werden miteinander vermischt und mit einer 30 µm Drahtrakel auf ein Rohpapier (50 g/m²) gestrichen und vorsichtig getrocknet. Das Beschichtungsgewicht beträgt 5,0 g m². Wird das beschichtete Papier mit einer erhitzten Nadel berührt, entsteht eine intensive blaue Farbe.

## Ansprüche

1. 2,2-Diarylchromenothiazole der allgemeinen Formel

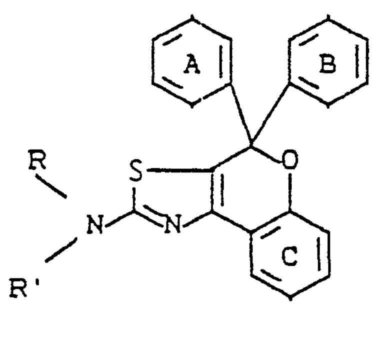

worin

R und R' unabhängig voneinander Wasserstoff, Alkyl, Cycloalkyl, Aralkyl bedeuten oder

R und R' ringgeschlossen sind und die Reste und die Ringe A, B und C ihrerseits in der Farbstoffchemie übliche nichtionische Substituenten tragen können, und mit einem Benzolring annelliert sein können.

2. 2,2-Diarylchromenothiazole gemäß Anspruch 1 der allgemeinen Formel

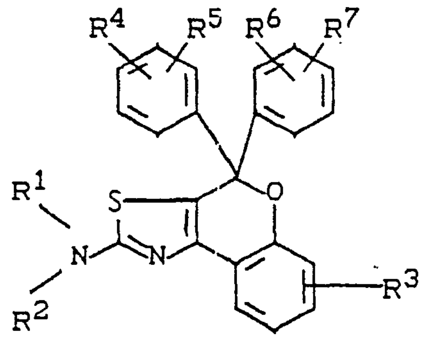

worin

$R^1$ und $R^2$ unabhängig voneinander Wasserstoff, $C_1$-bis $C_{22}$-Alkyl, das substituiert sein kann durch Halogen, Cyano, $C_1$-bis $C_{20}$-Alkoxycarbonyl, $C_1$-bis $C_{20}$-Alkoxy, Hydroxy, Amino, das seinerseits durch ein oder zwei $C_1$-bis $C_{20}$-Alkyl-, Phenyl-oder Benzylgruppen substituiert sein kann, Cyclohexyl, Phenyl, Benzyl oder Phenylethyl, die substituiert sein können durch Halogen, Hydroxy, $C_1$-bis $C_{18}$-Alkyl, $C_1$-bis $C_{18}$-Alkoxy, Phenoxy, Benzyloxy, Phenyl, Biphenyl, $C_1$-bis $C_{18}$-Alkylsulfonyl, Phenylsulfonyl, Cyano oder Amino, das seinerseits durch 1 oder 2 $C_1$-bis $C_{18}$-Alkyl, Phenyl oder Benzyl substituiert sein kann.

oder

$NR^1R^2$ einen Pyrrolidin-, Pyrazolin-, Piperidin-, Piperazin-oder Morpholinrest, die durch $C_1$-bis $C_4$-Alkyl oder Phenyl substituiert sein können,

$R^3$ Wasserstoff, $C_1$-bis $C_{22}$-Alkyl, Cyclohexyl, Nitro, gegebenenfalls durch $C_1$-bis $C_{12}$-Alkyl, $C_1$-bis $C_4$-Alkoxy oder Chlor substituiertes Phenyl oder Benzyl,

$R^4$ und $R^6$ unabhängig voneinander Wasserstoff, $C_1$-bis $C_{22}$-Alkyl, $C_1$-bis $C_{22}$-Alkoxy oder Halogen,

$R^5$ und $R^7$ unabhängig voneinander Wasserstoff, $C_1$-bis $C_{22}$-Alkyl, $C_1$-bis $C_{22}$-Alkoxy, $C_1$-bis $C_{22}$-Alkylthio, Hydroxy, Halogen, $C_2$-bis $C_6$-Alkenyloxy, Phenyl, Phenoxy, Cyclohexyloxy, Benzyloxy, Phenylethoxy oder Phenylthio, sowie die genannten Ringe durch $C_1$-bis $C_4$-Alkyl, $C_1$-bis $C_4$-Alkoxy oder Halogen substituiert sein können, oder $-NR^1R^2$ bedeuten oder

$R^4$ oder $R^6$ zusammen mit dem Benzolring, an den sie gebunden sind, einen Naphthalinring darstellen.

3. 2,2-Diarylchromenothiazole gemäß Anspruch 1 der allgemeinen Formel

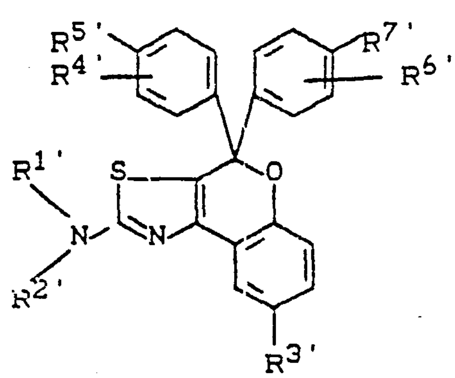

worin

$R^{1'}$ und $R^{2'}$ unabhängig voneinander Wasserstoff, $C_1$-bis $C_6$-Alkyl, das substituiert sein kann durch Cyano, Hydroxy, $C_1$-bis $C_3$-Alkoxycarbonyl oder $C_1$-bis $C_4$-Alkoxy, Benzyl oder Phenyl, die substituiert sein können durch Halogen, Hydroxy, $C_1$-bis $C_{12}$-Alkyl oder $C_1$-bis $C_4$-Alkoxy, Cyclohexyl, das durch $C_1$-bis $C_4$-Alkyl substituiert sein kann, oder

$NR^{1'}R^{2'}$ einen Pyrrolidin-, Pyrazolin-, Piperidin-, Piperazin-oder Morpholinrest, die durch Methyl

oder Phenyl substituiert sein können,

$R^{3'}$ Wasserstoff, $C_1$-bis $C_{12}$-Alkyl, Phenyl oder Benzyl,

$R^{4'}$ und $R^{6'}$ unabhängig voneinander Wasserstoff, $C_1$-bis $C_{12}$-Alkyl, $C_1$-bis $C_4$-Alkoxy oder Chlor und

$R^{5'}$ und $R^{7'}$ unabhängig voneinander Wasserstoff, $C_1$-bis $C_{12}$-Alkyl, $C_1$-bis $C_4$-Alkoxy, Phenoxy, Benzyloxy oder $-NR''R^{2'}$

bedeuten.

4. 2,2-Diarylchromenothiazole der Formel von Anspruch 3, worin

$R^{1'}$ für $C_1$-bis $C_6$-Alkyl,

$R^{2'}$ für $C_1$-bis $C_6$-Alkyl oder Cyclohexyl,

$R^{3'}$ für Wasserstoff oder Methyl,

$R^{4'}$ und $R^{6'}$ für Wasserstoff,

$R^{5'}$ für Methoxy, Di-$C_1$-bis $C_6$-alkylamino oder Cyclohexyl-$C_1$-bis $C_6$-alkylamino und

$R^{7'}$ für Di-$C_1$-bis $C_6$-alkylamino, Cyclohexyl-$C_1$-bis $C_6$-alkylamino oder Arylamino stehen.

5. Verfahren zur Herstellung der 2,2-Diarylchromenothiazole des Anspruchs 1, dadurch gekennzeichnet, daß man Thiazole der allgemeinen Formel

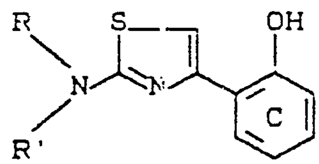

oder ihre Salze der allgemeinen Formel

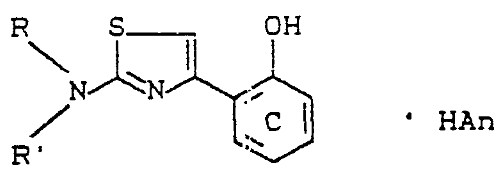

mit einer Carbinolverbindung der allgemeinen Formel

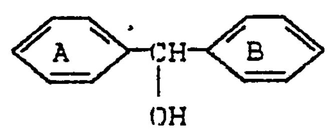

worin

R und R' die in Anspruch 1 angegebene Bedeutung haben,

A,B und C in der Farbstoffchemie übliche nichtionische Substituenten tragen können und

An für ein Anion steht,

umsetzt und oxidiert.

6. Aminothiazole der allgemeinen Formeln

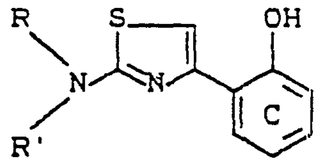

oder

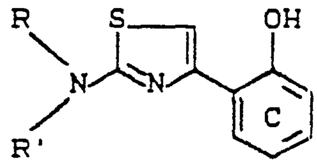

·HAn

worin

R und R' die in Anspruch 1 angegebene Bedeutung haben,

C in der Farbstoffchemie übliche nichtionische Substituenten tragen kann und

An für ein Anion steht.

7. Verfahren zur Herstellung der Aminothiazole des Anspruchs 6, dadurch gekennzeichnet, daß man Phenole der allgemeinen Formel

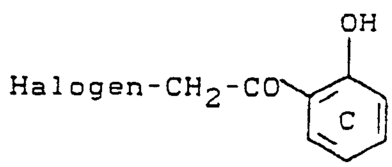

mit Thioharnstoffen der Formel

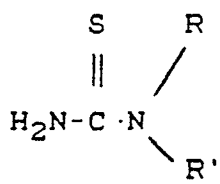

worin

R und R' die in Anspruch 1 angegebene Bedeutung haben und

C in der Farbstoffchemie übliche nichtionische Substituenten tragen kann,

umsetzt und das entstehende Halogenid gegebenenfalls durch ein anderes Anion austauscht.

8. Verwendung der 2,2-Diarylchromenothiazole gemäß Anspruch 1 als Farbbildner in einem druck- oder wärmeempfindlichen Aufzeichnungsmaterial.

9. Druck-oder wärmeempfindliches Aufzeichnungsmaterial, dadurch gekennzeichnet, daß es in seinem Reaktantensystem mindestens eine Verbindung des Anspruchs 1 als Farbbildner enthält.